# EUROPEAN PATENT SPECIFICATION

(11) **EP 0 418 237 B1**
(45) Date of publication and mention of the grant of the patent: **22.12.1993**
(21) Application number: 88907849.9
(22) Date of filing: 14.09.1988
(51) Int. Cl.: G01N 35/00, B01L 11/00

(54) **A METHOD FOR CONTINUOUSLY AND AUTOMATICALLY MAKING ANALYSES AND A DEVICE FOR PERFORMING THE METHOD**
VERFAHREN ZUR KONTINUIERLICHEN UND SELBSTTÄTIGEN DURCHFÜHRUNG VON ANALYSEN UND ANORDNUNG ZUR DURCHFÜHRUNG DES VERFAHRENS
PROCEDE SERVANT A EFFECTUER DES ANALYSES CONTINUELLEMENT ET AUTOMATIQUEMENT ET DISPOSITIF DE REALISATION DUDIT PROCEDE

(30) Priority: 15.09.1987 SE 8703563
(43) Date of publication of application: 27.03.1991
(73) Proprietor: LAMMERT, Jörgen, S-435 00 Mölnlycke (SE)
(72) Inventor: LAMMERT, Jörgen, S-435 00 Mölnlycke (SE)
(74) Representative: Roth, Ernst Adolf Michael
(86) International application number: SE8800477
(87) International publication number: WO8902601

(56) References cited:
- DE-A- 1 498 779
- DE-A- 1 523 059
- FR-A- 653 502
- US-A- 3 791 221
- US-A- 3 802 272

## Description

The present invention refers to a method for continuously and automatically carrying out analyses, e.g. dissolution tests, wherein substances at each test are supplied to at least one test vessel, in which the analysis is carried through under agitation, whereby between each test the used substances are poured out of the test vessel in that this is turned u0pside down, whereupon the agitating means and the test vessel are washed clean by means of at least one washing nozzle directed into the vessel, before a new test is carried through. The invention also refers to a device for performing the method.

### BACKGROUND OF THE INVENTION

For ascertaining a constant supply of a medical preparation to a patient during an extended period of time the pharmaceutical industry has developed tablets, capsules, etcetera, slowly emitting the active substance. In order to control in a laboratorical and uniform manner the manufactured product, there is a standardized equipment for determining emitting of an active component, prescribed i.a. in European and American pharmacopoeia. The method is named "dissolution test" and the apparatus consists of a vessel having a prescribed geometry and made of an inert, transparent material, and which vessel is immersed in a bath heated to 37°C. In the vessel is furthermore provided either an agitator or a rotating tablet basket, which have also prescribed geometry. The rotational speed of the agitator is held constant during the entire test period. The vessel is filled with a buffer solution, the volume of which is defined and the temperature of which is also held at 37°C. The preparations which shall be tested are introduced into the vessel or in the rotating basket and the buffer solution is analysed at determined time intervals regarding the content of active substance.

This gives a measure on the properties of the preparation regarding supply of active substance to the patient.

Standardized equipments for "dissolution test" incorporating up to 6 test vessels per unit are on the market since many years (Prolabo, Hansen, etcetera). These equipments however are intended for manual operation regarding emptying after concluded test, cleaning and filling up buffer solution and control of correct buffer solution at intended temperature. These operations are time-wasting and monotonous, which makes an automatization desirable.

Hansen therefore has introduced an automatic mechanism for filling up the buffer solution. Perkin-Elmer and Zymark use their laboratory robots for distribution of preparations to the test vessels and for taking out test samples for analysis. Zymark also has introduced a device for automatical emptying and cleaning of the the vessels, which device consists of a hose manipulated by a laboratory robot, and which hose with a pump empties the vessel. Cleaning liquid is thereupon pumped in and sucked out in several cycles and the buffer solution is finally filled up. When this has reached a temperature of 37°C the sucking hose is lowered by the robot to a level specific for every vessel and the excess is sucked off. This method for emptying and cleaning has proven itself to be uncertain, as residual tablets and capsules may have been left in the vessel or have caused clogging of hoses and valves.

There is also known a method and a device for continuous automatic analysis of solid test samples according to DE-A-15 23 059, which device incorporates a number of test vessels, which in level with the vessel bottom are provided with a pivot shaft, about which the test vessel may be swung to an emptying position thus that the content may be emptied in a discharge hopper. In the hopper is also provided a washing nozzle, which can wash clean the interior of the thus swung down test vessel. The design implies, that the agitating means, if such are used, will not be cleaned between different tests, and therefore will contaminate the new test solution. Another drawback is that the structure will not allow the test vessels to be immersed in a tempered bath, whereby rather big temperature variations may occur. The cleaning effect is neither quite satisfactory as residual tablets and the the like has a tendency to stick very firmly to the vessel walls.

### PURPOSE AND MOST ESSENTIAL FEATURES OF THE INVENTION

The purpose of the invention is to provide a reliable, automatical method and a device for emptying the test vessel and a careful cleaning of this and of its agitating means and the filling and washing nozzels, which may be immersed in the vessel. The cleaning must be 100% effective thus that the manual control is entirely eliminated. The design shall be simple, with a minimum of movable parts and permit that analyses can be made all around the clock. These tasks have been solved in that the agitating means as well as the washing nozzle participate in the emptying movement of the test vessel, and that the agitating means and the washing nozzle are activated during and/or after the emptying of the test vessel, and the device for performing the method at automatic analysis apparatuses, which incorporates at least one test vessel adapted to house test substances, which for emptying is pivotable about a mainly horizontal shaft, such that the test substances are discharged, and which apparatus is provided with at least one agitating means and at least one washing nozzle arranged in the test vessel, is characterized in that the agitating means and its driving device and the washing nozzle with its holder are arranged to participate in the emptying motion of the test vessel, and that the agitating means and the washing nozzle are activatable during the pivoting movement of the test vessel.

### DESCRIPTION OF THE DRAWINGS

The invention hereinafter will be further described with reference to an embodiment shown in the accompanying drawings.

Fig. 1 shows the analysis apparatus according to the invention in a side view and with the test vessel in position for analysis.

Fig. 2 shows also a side view of the analysis apparatus according to Fig. 1 with the test vessel in a swung up cleaning and washing position.

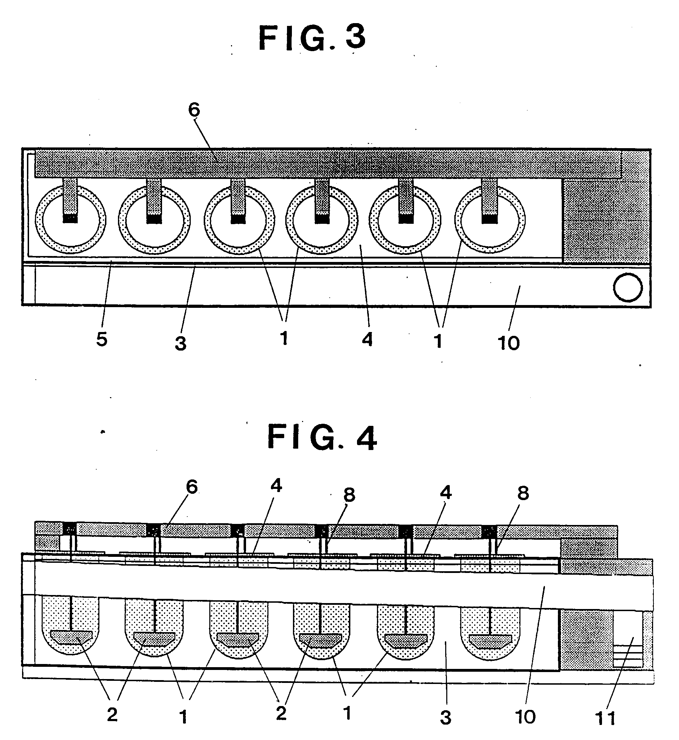

Fig. 3 shows the analysis apparatus according to Fig. 1 in a view from above.

Fig. 4 shows the analysis apparatus according to Fig. 3 in a front view.

### DESCRIPTION OF EMBODIMENT

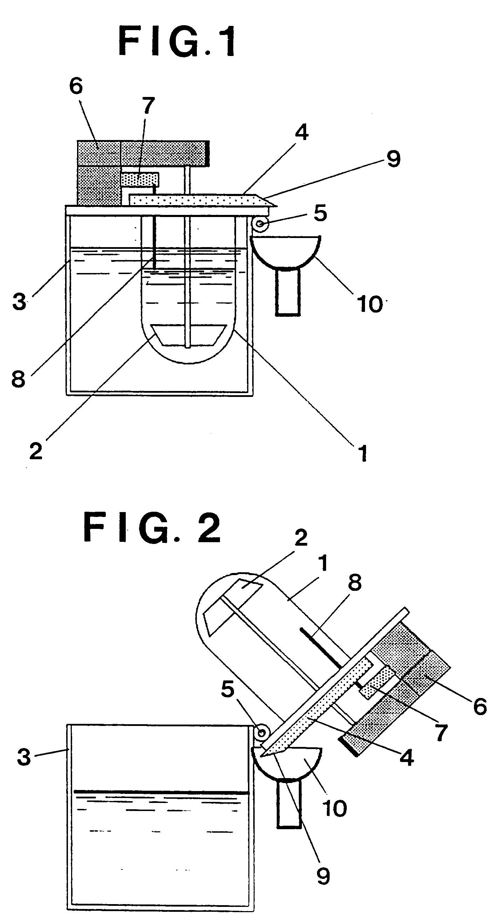

The analysis apparatus according to the invention consists of a test vessel 1, preferably of transparent material, e.g. glass, in which is immersed an agitating means 2, which can be wings, a table basket or the like. The test vessel in its analysis position is immersed in an outer vessel 3 filled with a thermostatically controlled bath for maintaining a constant temperature on the substance, which is introduced in the vessel 1. The outer vessel 3 and the test vessel 1 are both provided with a common cover 4, which is pivotably supported at the upper edge of the outer vessel 3 about a horizontal shaft 5. On the cover is fixedly arranged a driving device 6 for the agitating means and a holder 7 for a combined filling tube and washing nozzle 8, which latter is immersed in the test vessel 1. At one end edge of the cover 4 is provided a discharge opening 9 just in front of the pivot shaft 5, through which opening the content of the test vessel is emptied in a discharge hopper 10 or a gutter, when the test vessel is in the cleaning and washing position shown in Fig. 2.

In the analysis position shown in Fig. 1, the test vessel 1 is supplied with, e.g. a buffer solution, in a somewhat bigger volume than that prescribed for the test. When the solution has obtained the correct temperature, the excess is sucked off to a level calibrated for the vessel. The substance to be tested, e.g. a tablet, a capsule or the like is thereupon positioned in the vessel, and this can be made either manually or automatically by means of a robot, a feeder or the like. After completed test, during which the agitating means rotates at a constant speed, the content is poured out of the test vessel, during which emptying motion the agitating means preferably continue to work, thus that any sedimentation of heavier particles in the test substance is avoided. The discharge opening 9 of the cover 4 thereby shall be so big that residual tablets or the like can pass freely. With the test vessel still in cleaning position according to Fig. 2 the inner side of the vessel and the agitating means is cleaned by washing with one or more rinsing agents, which under pressure is introduced through the combined filling and washing nozzle 8. The last rinsing is made with buffer solution, whereupon the vessel is returned to analysis position according to Fig. 1 and a new test cycle is initiated.

Several test vessels are preferably arranged in the same outer vessel 3, such as shown in Fig.s 3 and 4. The test vessels may also be pivotable individually and independent of each other or they may be emptied together by a single driving device 11 common for all test vessels.

## Claims

1. A method for continuously and automatically carrying out analyses, e.g. dissolution tests, wherein substances at each test are supplied to at least one test vessel (1), in which the analysis is carried through under agitation, whereby between each test the used substances are poured out of the test vessel in that this is turned upside down, whereupon the agitating means (2) and the test vessel are washed clean by means of at least one washing nozzle (8) directed into the vessel, before a new test is carried through,
**characterized therein**,
that the agitating means (2) as well as the washing nozzle (8) participate in the emptying movement of the test vessel (1), and that the agitating means and the washing nozzle are activated during and/or after the emptying of the test vessel.

2. A device for performing the method according to claim 1 at automatic analysis apparatuses, which incorporates at least one test vessel (1) adapted to house test substances, which for emptying is pivotable about a mainly horizontal shaft (5), such that the test substances are discharged, and which apparatus is provided with at least one agitating means (2) and at least one washing nozzle (8) arranged in the test vessel,
**characterized therein**,
that the agitating means (2) and its driving device (6) and the washing nozzle (8) with its holder (7) are arranged to participate in the emptying motion of the test vessel (1), and that the agitating means (2) and the washing nozzle (8) are activatable during the pivoting movement of the test vessel.

3. A device as claimed in claim 2,
**characterized therein**,
that the test vessel (1) is equipped with a cover (4), on which are mounted the agitating means (2), its driving device (6) and the washing nozzle (8).

4. A device as claimed in claim 3,
**characterized therein**,
that the cover (4) at one side edge is pivotably supported about said shaft (5), and that the cover (4) at said side edge is provided with a discharge opening (9).

5. A device as claimed in claim 4,
**characterized therein**,
that the test vessel (1) in analysis position is situated inside an outer vessel (3) and that the pivot shaft (5) of the test vessel (1) is fitted to the upper edge of the outer vessel.

6. A device as claimed in claim 4,
**characterized therein**,
that below the pivot shaft (5) for the cover (4) is provided a discharge hopper or gutter (10).

7. A device as claimed in one or more of claims 2 - 6,
**characterized therein**,
that several test vessels (1) are arranged in a row beside each other and which are dischargeable together or alternatively separately.

## Patentansprüche

1. Methode zur kontinuierlichen und automatischen Durchführung von Analysen, z.B. von Zersetzungstests, wobei Substanzen bei jedem Test in mindestens ein Testgefäß (1) eingefüllt werden, in dem die Analyse unter Rühren durchgeführt wird, wobei zwischen jedem Test die verwendeten Substanzen aus dem Testgefäß in der Weise ausgegossen werden, daß das Testgefäß mit der Unterseite nach oben gedreht wird, woraufhin das Rührmittel (2) und das Testgefäß mittels mindestens einer Waschdüse (8) ausgewaschen werden, die in das Gefäß gerichtet wird, bevor ein neuer Test durchgeführt wird,
**gekennzeichnet dadurch**,
daß das Rührmittel (2) wie auch die Waschdüse (8) an der Entleerungsbewegung des Testgefäßes (1) teilnehmen, und daß das Rührmittel und die Waschdüse während und/oder nach der Entleerung des Testgefäßes betätigt werden.

2. Eine Vorrichtung zur Ausführung der Methode nach Anspruch 1 an automatischen Analyseapparaten, wobei die Vorrichtung mindestens ein Testgefäß (1) zur Aufnahme der Testsubstanzen aufweist, das zur Entleering um eine im wesentlichen horizontale Welle (5) drehbar angebracht ist, um dadurch die Testsubstanzen auszugießen, und wobei der Apparat mit mindestens einem Rührmittel (2) und mindestens einer Waschdüse (8) versehen ist, die im Testgefäß angebracht sind,
**gekennzeichnet dadurch**,
daß das Rührmittel (2) und seine Antriebsvorrichtung (6) und die Waschdüse (8) mit ihrer Halterung (7) so angebracht sind, daß sie an der Entleerungsbewegung des Testgefäßes (1) teilnehmen, und dadurch, daß das Rührmittel (2) und die Waschdüse (8) während der Drehbewegung des Testgefäßes betätigt werden können.

3. Eine Vorrichtung nach Anspruch 2,
**gekennzeichnet dadurch**,
daß das Testgefäß (1) mit einer Abdeckung (4) versehen ist, an der das Rührmittel (2), seine Antriebsvorrichtung (6) und die Waschdüse (8) angebracht sind.

4. Eine Vorrichtung nach Anspruch 3,
**gekennzeichnet dadurch**,
daß die Abdeckung (4) an einem seitlichen Rand drehbar auf der Welle (5) aufliegt und daß die Abdeckung (4) an diesem seitlichen Rand mit einer Ausflußöffnung (9) versehen ist.

5. Eine Vorrichtung nach Anspruch 4,
**gekennzeichnet dadurch**,
daß das Testgefäß (1) sich in der Analyseposition innerhalb eines äußeren Gefäßes (3) befindet und daß die Drehwelle (5) des Testgefäßes (1) am oberen Rand des Außengefäßes angebracht ist.

6. Eine Vorrichtung nach Anspruch 4,
**gekennzeichnet dadurch**,
daß unterhalb der Drehwelle (5) für die Abdeckung (4) ein Ausflußtrichter oder -rohr (10) angebracht ist.

7. Eine Vorrichtung nach einem der Ansprüche 2 - 6,
**gekennzeichnet dadurch**,
daß mehrere Testgefäße (1) in einer Reihe nebeneinander angeordnet sind, die gemeinsam oder als Alternative einzeln entleerbar sind.

## Revendications

1. Un procédé pour réaliser de façon continue et automatiquement des analyses, par exemple, des essais de dissolution, dans lequel les substances à chaque essai sont amenées à au moins un récipient d'essai (1) dans lequel l'analyse est mise en oeuvre sous agitation si bien qu'entre chaque essai les substances utilisées sont déversées du récipient d'essai car il est retourné, après quoi le moyen d'agitation (2) et le récipient d'essai sont lavés au moyen d'au moins une buse de lavage (8) dirigée dans le récipient, avant la mise en oeuvre d'un nouvel essai, caractérisé en ce que le moyen d'agitation (2) ainsi que la buse de lavage (8) participent au vidage du récipient d'essai (1) et en ce que le moyen d'agitation et la buse de lavage sont activés pendant et/ou après le vidage du récipient d'essai.

2. Un dispositif pour mettre en oeuvre le procédé selon la revendication 1 avec des appareils d'analyse automatiques qui comprend au moins un récipient d'essai (1) agencé pour loger les substances d'essai, qui pour le vidage est susceptible de pivoter autour d'un axe essentiellement horizontal (5) de telle façon que les substances d'essai sont déchargées et cet appareil est muni d'au moins un moyen d'agitation (2) et d'au moins une buse de lavage (8) disposés dans le récipient d'essai, caractérisé en ce que le moyen d'agitation (2) et son dispositif d'entraînement (6) et la buse de lavage (8) avec son support (7) sont disposés pour participer au vidage du récipient d'essai (1) et en ce que le moyen d'agitation (2) et la buse de lavage (8) sont susceptibles d'être activés pendant le mouvement de pivotement du récipient d'essai.

3. Un dispositif selon la revendication 2, caractérisé en ce que le récipient d'essai (1) est muni d'un couvercle (4) sur lequel sont montés le moyen d'agitation (2), son dispositif d'entraînement (6) et la buse de lavage (8).

4. Un dispositif selon la revendication 3, caractérisé en ce que le couvercle (4) sur un bord latéral est supporté de façon pivotante autour dudit axe (5) et en ce que le couvercle (4) sur ledit bord latéral est muni d'une ouverture de décharge (9).

5. Un dispositif selon la revendication 4, caractérisé en ce que le récipient d'essai (1) dans la position d'analyse est situé à l'intérieur du récipient extérieur (3) et en ce que l'axe de pivotement (5) du récipient d'essai (1) est adapté sur le bord supérieur du récipient extérieur.

6. Un dispositif selon la revendication 4, caractérisé en ce qu'au-dessous de l'axe de pivotement (5) pour le couvercle (4), il est prévu une trémie de décharge ou gouttière (10).

7. Un dispositif selon l'une quelconque des revendications 2 à 6, caractérisé en ce que les divers récipients d'essai (1) sont disposés selon une rangée les uns par rapport aux autres et ils sont susceptibles d'être déchargés ensemble ou alternativement séparément.
